# EUROPEAN PATENT SPECIFICATION

(11) **EP 2 266 543 B1**
(45) Date of publication and mention of the grant of the patent: **07.08.2013**
(21) Application number: 10011678.9
(22) Date of filing: 06.06.2003
(51) Int. Cl.: A61K 35/00, A61K 48/00, A61K 9/50, A61K 9/00, A61P 19/00

(54) **Non-polymeric hematopoietic cell clots for delivery of active agents**
Nichtpolymere hämatopoetische Zellgerinnsel für die Abgabe von Wirkstoffen
Amas cellulaires hématopoietiques non-polymèriques pour la distribution d'agents actifs

(30) Priority: 06.06.2002 US 386870 P
(43) Date of publication of application: 29.12.2010
(62) Divisional of application: 03757356.5
(73) Proprietor: The Brigham and Women's Hospital, Inc., Boston MA 02115 (US)
(72) Inventor: Pascher, Arnulf, 8042 Graz (AT); Palmer, Glyn, Allston, MA 02134 (US); Ghivizzani, Steven, Gainesville, Florida 32610-0137 (US); Evans, Christopher, Cohasset MA 02025 (US)
(74) Representative: Schrell, Andreas

(56) References cited:
- WO-A1-02/068010
- US-A- 4 902 288
- GOTO H ET AL: "Transfer of LacZ marker gene to the meniscus", JOURNAL OF BONE AND JOINT SURGERY. AMERICAN VOLUME, JOURNAL OF BONE AND JOINT SURGERY, US, vol. 81, no. 7, 1 January 1999 (1999-01-01), pages 918-925, XP002971193, ISSN: 0021-9355
- BONADIO J: "Tissue engineering via local gene delivery", JOURNAL OF MOLECULAR MEDICINE, SPRINGER VERLAG, DE, vol. 78, 1 January 2000 (2000-01-01), pages 303-311, XP002971194, ISSN: 0946-2716, DOI: DOI:10.1007/S001090000118
- PASCHER A ET AL: "Gene delivery to cartilage defects using coagulated bone marrow aspirate.", GENE THERAPY JAN 2004 LNKD- PUBMED:14712297, vol. 11, no. 2, January 2004 (2004-01), pages 133-141, XP002614554, ISSN: 0969-7128

## Description

### FIELD OF THE INVENTION

This invention relates to active agent delivery, and more particularly, to a non-polymeric hematopoeitic cell clot to aid in the delivery of an active agent.

### BACKGROUND OF THE INVENTION

The treatment of cartilage, bone, vertebral disc, and soft tissue lesions with biological factors and cells is an emerging approach for the enhancement of defect repair. The administration of recombinant proteins and protein growth factors to encourage tissue regrowth, leads to disappointing results as the maintenance of therapeutic concentrations requires very high loading doses or repeat administration, thereby decreasing the efficiency of repair, while increasing the cost, complexity, and the risk of generating unwanted side effects from exposure of non-target organs.

One approach designed to facilitate the application of recombinant proteins to tissue repair has been to incorporate them into a biocompatible matrix or slow release device for implantation into a tissue defect, thereby localizing the proteins to the site of damage and possibly provide a three-dimensional structure for emigrating cells to colonize. Matrices that have been evaluated for repair of musculoskeletal tissues include a variety of synthetic and natural polymers. These systems also have limitations in that the proteins loaded into the matrix can be extraordinarily expensive to produce in quantity, rarely have prolonged and uniform releases, while the newly forming repair tissue can be adversely influenced by the presence of a foreign, implanted material. Gene transfer offers an approach that may overcome the many limitations of protein delivery to damaged tissues (Bonadio et al., 1999; Evans and Robbins, 1995; Evans et al., 1995; Kang et al., 1997; Smith et al., 2000).

US 4,902,288 discloses an implantable immunotherapy system comprising a matrix having immunologically active cells incorporated therein. Goto et al., (J. Bone and Joint Surg. 81(7):918-925, 1999) disclose the treatment of meniscal damage by transforming genes that encode growth factors. WO 02/068010 A I which was published after the priority date of the present invention shows a composite bone marrow graft material having an enriched population of uniformly distributed progenitor cells.

### SUMMARY OF THE INVENTION

The invention presents a novel system for the application of active substances, such as gene delivery vehicles, cells and soluble proteins for the healing of damaged tissues. It has been discovered that the use of non-polymeric hematopoeitic cell clots can be used to deliver a substance into a subject. The non-polymeric hematopoeitic cell clot functions as a delivery vehicle for the substance into the subject.

According to one aspect, the invention is a method of delivering a substance to a subject. The method comprises administering to a subject a non-polymeric hematopoeitic cell clot containing a substance to deliver the substance to the subject.

Also disclosed is a method of preparing a non-polymeric hematopoeitic cell clot substance delivery system. This method comprises adding a substance to a sample of hematopoeitic cells and allowing the sample of hematopoeitic cells containing the substance to form a non-polymeric hematopoeitic cell clot.

According to yet another aspect, the invention is a substance delivery system comprising a non-polymeric hematopoeitic cell clot having a substance incorporated therein according to claims 1 and 2.

The non-polymeric hematopoeitic cell clot comprises bone marrow cells. Disclosed are also blood cells or any other type of cell that would form a clot. The substance may comprise a gene transfer vehicle, additional cells, such as genetically engineered cells or naive cells, proteins, such as recombinant or soluble proteins, bioactive molecules or any other type of substance that could affect a subject.

The non-polymeric hematopoeitic cell clot maybe delivered into any type of tissue. For instance, in some embodiments the tissue is bone, soft tissues, cartilage, ligaments, tendons, meniscuses, and invertebral disks or any other region of the body.

The shape and size of the non-polymeric hematopoeitic cell clot in some embodiments may be determined by a vessel. The non-polymeric hematopoeitic cell clot may be homogenized with the substance. In other embodiments the non-polymeric hematopoeitic cell clot may be genetically modified to express at least one of growth factors and other gene products that facilitate tissue repair.

The non-polymeric hematopoeitic cell clot in other embodiments may have a volume that is determined by the size of a tissue to be repaired.

In yet other embodiments the non-polymeric hematopoeitic cell clot can be collected from a subject. The bone mass cells may in other embodiments be harvested from iliac crests, from osteochondral defects that expose underlying bone marrow or any other area of a subject from which bone marrow cells could be harvested.

The substrate may optionally be in the form of a solution.

The non-polymeric hematopoeitic cell clot containing the substance may be titrated. The titration may be performed using a pipette.

In some embodiments the non-polymeric hematopoeitic cell clot is mixed with a suspension of at least one naïve and genetically modified cells, forming a cell suspension. The cell suspension may contain additional gene vectors or no additional gene vectors.

In other embodiments the delivery may be a slow, localized release of the substance from the non-polymeric hematopoeitic cell clot. The non-polymeric hematopoeitic cell clot may be shaped in a way to allow an effective delivery of the substance. The substance delivery system may result in the regeneration of tissue in the area of substance delivery.

The non-polymeric hematopoeitic cell clot may, in some embodiments be produced from a sample of hematopoeitic cells that is allowed to clot for 15-30 minutes, at room temperature or when placed in a vessel. The non-polymeric hematopoeitic cell clot may then be harvested from the vessel.

The non-polymeric hematopoeitic cell clot also may be produced from a sample of hematopoeitic cells which is washed in a phosphate buffer saline. Any unbound substance can be removed from the non-polymeric hematopoeitic cell clot.

According to other embodiments the non-polymeric hematopoeitic cell clot can be implanted into a subject. The substance may be delivered into the subject. The delivery may be a slow, localized release of the substance from the non-polymeric hematopoeitic cell clot. Optionally, the non-polymeric hematopoeitic cell clot delivery system can be used to regenerate tissue.

The sample of hematopoeitic cells may be collected from a subject or may be obtained from another source of hematopoietic cells. The subject may be the same or a different subject into which the clot is later implanted.

### BRIEF DESCRIPTION OF THE DRAWINGS

Various embodiments of the present invention will now be described, by way of example, with reference to the accompanying drawings, in which:
Fig. 1 is an exemplary list of genes that have been used in gene therapy;
Fig. 2 is a graph of the loading capacity of a human blood clot and a collagen-glycosaminoglycan-matrix;
Fig. 3 is a picture of a human blood clot with pre-infected rabbit bone marrow cells 24 hours after clotting;
Fig. 4 is a picture of a 2 mm thick, 30 mm diameter human blood clot (formed in a tissue culture well);
Fig. 5 is a picture of GFP positive cells in clots at day 1 (Fig. 5a) and day 21 (Fig. 5b);
Fig. 6 is a graph of the production of TGF-β by rabbit blood clots containing Ad TGF-β infected rabbit bone marrow cells;
Fig. 7 is a graph of the expression of TGF-β to surrounding media, wherein "BL" stands for blood and "BM" stands for bone marrow;
Fig. 8 is a graph of the expression of TGF-β in disaggregated rabbit bone marrow and blood clots;
Fig. 9 is a graph of the stability of the adenovirus in a clot;
Fig. 10 is a graph of the in vivo gene expression in rabbits at day 3;
Fig. 11 is a picture of rabbit bone marrow clot after 6 weeks in vitro, using Gomori's Trichrome Kit staining; and
Fig. 12 is a picture of rabbit bone marrow clot with Ad TGF-β after 6 weeks in vitro, using Gomori's Trichrome Kit staining.

### DETAILED DESCRIPTION

According to the present invention, it was discovered that a substance could be delivered to a subject by administering to the subject a non-polymeric hematopoeitic cell clot containing the substance. Prior art methods for delivering a substance to a subject, have many limitations. For instance, some of them are expensive, complex and rarely have desired sustained and uniform releases.

As used herein a "hematopoeitic cell clot" is a clot comprising bone marrow cells that can form a clot under various conditions. Examples are bone marrow clots. Aspirates of bone marrow can easily be obtained from a subject using minimally invasive procedures. This is in contrast to the manufacture of artificial matrices which is much more time-consuming, expensive and labor intensive. To generate bone marrow clots a suitable volume of bone marrow aspirates can be harvested from sources rich in bone marrow such as the iliac crests, from osteochondral defects that expose the underlying bone marrow or other appropriate sites. Bone marrow aspirates and blood generally are of the same consistency and have similar coagulation properties.

The use of bone marrow clots in tissue repair offers the advantage that the formation of blood clots and the migration of bone marrow cells are part of the natural repair response following generation of osteochondral defects, bone, tendon, meniscus or intervertebral disk defects. In addition, bone marrow clots are enriched with stem cells, which retain the capacity to form the different tissues of the body; hence, the clot represents the natural microenvironment for a repair response. If coupled with the appropriate biological agents, the hematopoeitic clot has the potential to promote repair of several tissue types.

The hematopoeitic cell may be isolated from the same subject into whom the clot will be delivered, from another subject into whom the substance will not be delivered, from a lab sample grown *in vitro* or from any other source of hematopoeitic cells. Clearly, different situations and substances would favor different sources from which the hematopoeitic cell clot would be taken. For example, if the hematopoeitic cell clot is obtained from the same subject into which the substance will be delivered, the clot is completely natural and autologous to the subject. Therefore, the hematopoeitic cells are less likely to interfere with the substance delivery, inhibit the substance's desired effect or produce an immune response.

The hematopoeitic cell clot can have any size and shape. For instance, the hematopoeitic cell clot may be used in whatever form it naturally takes during the clotting process. Alternatively steps may be taken to form the hematopoeitic cell clot into a specific size or shape. A hematopoeitic cell clot of a specific size or shape may be useful for repair of a specific tissue defect. In that case it may be desirable to produce a clot having a size similar to the particular defect being corrected or treated.

One method for preparing a hematopoeitic cell clot in a specific size or shape is to use a molding vessel. For instance, the hematopoeitic cell clot can be formed in a vessel; so that the sample of hematopoeitic cells and substance mixture will solidify in the vessel. In such a manner, the clot will have a size and shape which is determined by the vessel's size and shape. The hematopoeitic cell clot may also be shaped in a way to allow effective delivery of a substance, such as a drug, i.e. even in the absence of a tissue defect. The solid state of the hematopoeitic cell clot allows the clot to be easily handled and implanted at sites of damage.

As mentioned above, the hematopoeitic cell clot may be useful for the repair of defective tissue. The clot can be placed into the tissue to help in the healing process. Preferably a substance that is also helpful in the repair process is incorporated into the clot. It is possible in some instances that the clot may be used alone to simply provide a matrix for ingrowth of cells during the repair process, but preferably a substance, such as a cell, drug or gene vector, is incorporated therein. The defective tissue may be any tissue in need of repair. For instance the tissue may be bone and various soft tissues, including but not limited to cartilage, ligaments, tendons, meniscuses and intervertebral disks. Alternatively, the hematopoeitic cell clot can be used as an in vitro system to engineer or repair tissues for subsequent implantation. For in vitro tissue formation, the hematopoeitic cell clots can be seeded with the cells, and cultured in the appropriate media.

The hematopoeitic cell clot may also be used to deliver drugs or cells to a subject in the absence of any tissue to be repaired. For instance the clot may be used as any other sustained release device is used to deliver a compound to a subject. The specific uses will depend on the type of drug, cell or gene vector being delivered to the subject.

As used herein a "subject" is a vertebrate such as a human, non-human primate, cow, horse, pig, sheep, goat, dog, cat, or rodent.

The formation of the hematopoeitic cell clot can occur under various conditions, such as at room temperature. For example, the coagulation of rabbit or human blood and bone marrow aspirate will occur within approximately 15-30 minutes. This clot may therefore be generated and implanted intra-operatively, if it is so desired.

Once the hematopoeitic cell clot has formed, any unbound substance may be removed from the clot. For example, the hematopoeitic cell clot could be washed in a solution such as a phosphate buffer saline. Examples of more detailed methods for preparing the clots are set forth in the description of experiments presented below. Those of ordinary skill in the art are aware of other methods for preparing clots with hematopoeitic cells.

As used herein a "non-polymeric hematopoeitic cell clot" is a hematopoetic cell clot, as defined above, wherein a polymer matrix is not incorporated into the clot or used as the structure for the clot. Most drug-delivery devices for tissue repairs use a polymer matrix as a structure for delivering the drug. A polymer matrix is formed from polymers, such as modified or natural polysaccharides, such as chitosan, chitin, hyaluronan, glycosaminoglycan, chondroitin sulfate, keratan sulfate, dermatan sulfate, heparin, or heparin sulfate. A polymer may be a natural, recombinant or synthetic protein, such as soluble collagen or soluble gelatin, or a polyamino acids, such as for example a polylysine. A polymer may also be polylactic acid, polyglycolic acid, a synthetic homo and block copolymers containing carboxylic, amino, sulfonic, phosphonic, phosphenic functionalities with or without additional functionalities such as for example without limitation hydroxyl, thiol, alkoxy, aryloxy, acyloxy, and aroyloxy. Additionally, a polymer may comprise orthoesters, anhydrides, propylene-co-fumarates, or a polymer of one or more alpha-hydroxy carboxylic acid monomers, (e.g. alpha-hydroxy acetic acid (glycolic acid) and/or alpha-hydroxy propionic acid (lactic acid)).

A polymer may be initially dissolved or suspended in a buffer containing inorganic salts such as sodium chloride, potassium calcium, magnesium phosphate, sulfate, and carboxylate. A polymer may also dissolved or suspended in a buffer containing an organic salt such as glycerol-phosphate, fructose phosphate, glucose phosphate, L-Serine phosphate, adenosine phosphate, glucosamine,galactosamine, HEPES, PIPES, and MES.

A substance is incorporated into the non-polymeric hematopoeitic cell clot. As used herein a "substance" is any composition that will have an effect on a subject, including a diagnostic effect. The substance, may be, for example, a cell or any other active agent, e.g. a drug or a gene vector capable of expressing a peptide, a small molecule, etc. The substance is an exogenous substance. That is, it is one that is added to the sample of hematopoeitic cells and was not present in the cell sample before it was taken from its prior environment (i.e. a subject, an *in* vitro environment, etc.). Examples of the substance are gene transfer vehicles (viral and non-viral), additional cells, genetically engineered or naïve, recombinant, soluble or any other type of proteins or other bioactive molecules, such as growth factors.

An active agent as used herein is any compound which has a diagnostic, prophylactic, or therapeutic effect in a biological organism. Active agents include compounds such as proteins, peptides, antibodies, polysaccharides, nucleic acids (e.g. RNA, DNA, PNA, multiplexes of them (e.g. triplex)), saccharides, glycoproteins, amino acids, viruses, heterogeneous mixtures of macromolecules (e.g. a natural product extract) and hybrid macromolecules (e.g. protein/nucleic acid hybrids, albumin conjugated proteins, drugs with linkers inorganic molecules, organic molecules, or combinations thereof).

A bioactive agent is any compound which has a prophylactic or therapeutic effect in a biological organism. In some embodiments the bioactive agent is any of the following agents: adrenergic agent; adrenocortical steroid; adrenocortical suppressant; agents for treating cognition, antiplatelets, aldosterone antagonist; amino acid; anabolic; analeptic; analgesic; anesthetic; anorectic; anti-acne agent; anti-adrenergic; anti-allergic; anti-Alzheimer's, anti-amebic; anti-anemic; anti-anginal; anti-arthritic; anti-asthmatic; anti-atherosclerotic; antibacterial; anticholinergic; anticoagulant; anticonvulsant; antidepressant; antidiabetic; antidiarrheal; antidiuretic; anti-emetic; anti-epileptic; antifibrinolytic; antifungal; antihemorrhagic; antihistamine; antihyperlipidemia; antihypertensive; antihypotensive; anti-infective; anti-inflammatory; antimicrobial; antimigraine; antimitotic; antimycotic, antinauseant, antineoplastic, antineutropenic, antiparasitic; antiproliferative; antipsychotic; antirheumatic; antiseborrheic; antisecretory; antispasmodic; antithrombotic; anti-ulcerative; antiviral; anxiolytics, appetite suppressant; blood glucose regulator; bone resorption inhibitor; bronchodilator; cardiovascular agent; cholinergic; COX1 inhibitors, COX2 inhibitors, direct thrombin inhibitors, depressant; diagnostic aid; diuretic; dopaminergic agent; estrogen receptor agonist; fibrinolytic; fluorescent agent; free oxygen radical scavenger; gastrointestinal motility effector; glucocorticoid; GPIIbIIIa antagonists, hair growth stimulant; hemostatic; histamine H2 receptor antagonists; hormone; human growth hormone, hypocholesterolemic; hypoglycemic; hypolipidemic; hypnotics, hypotensive; imaging agent; immunological agents such as immunizing agents, immunomodulators, immunoregulators, immunostimulants, and immunosuppressants; keratolytic; LHRH agonist; mood regulator; mucolytic; mydriatic; nasal decongestant; neuromuscular blocking agent; neuroprotective; NMDA antagonist; non-hormonal sterol derivative; plasminogen activator; platelet activating factor antagonist; platelet aggregation inhibitor; proton pump inhibitors, psychotropic; radioactive agent; scabicide; sclerosing agent; sedative; sedative-hypnotic; selective adenosine Al antagonist; serotonin antagonist; serotonin inhibitor; serotonin receptor antagonist; statins, steroid; thyroid hormone; thyroid inhibitor; thyromimetic; tranquilizer; amyotrophic lateral sclerosis agent; cerebral ischemia agent; Paget's disease agent; unstable angina agent; vasoconstrictor; vasodilator; wound healing agent; xanthine oxidase inhibitor.

One preferred use of the non-polymeric hematopoeitic cell clot is to repair bone and tissue defects. Proteins that are most likely linked to cartilage, bones and soft tissue repair are the members of the transforming growth factor - β (TGF-β) super family including TGF-β S 1-3, various bone morphogenetic proteins (BMPs), fibroblast growth factors, growth hormone, and insulin-like growth factors (IGFs).

The in vivo administration of recombinant proteins to enhance the formation of cartilage and cartilage repair as well as that of bone and soft tissue has been investigated in various defect models and experimental animals.² Despite promising results, the clinical application of recombinant proteins is hindered by the short biological half lives of these molecules and lack of an effective method for sustained, target delivery. Direct injection of protein growth factors into ² Hunziker, 2001; Nixon et al., 1999; Sellers et al., 1997 sites of tissue damage has led to disappointing results because the factors are diluted by body fluids, quickly metabolized or disseminated to other tissues. Thus, the maintenance of therapeutic concentrations requires very high loading doses or repeat administration. This decreases the efficiency of repair, while increasing the costs, complexity, and risk of generating unwanted side effects from exposure of non-target organs. The non-polymeric hematopoeitic cell clots described herein overcome many of these problems, as demonstrated in the examples presented below.

The clot is also useful for delivering genes to a subject, generally or to a specific tissue of a subject. As used herein, a "gene" is an isolated nucleic acid molecule of greater than thirty nucleotides, more typically one hundred nucleotides or more, in length. It generally will be under the control of an appropriate promoter, which may be inducible, repressible, or constitutive. Any genes that would be useful in replacing or supplementing a desired function, or achieving a desired effect such as the inhibition of tumor growth, could be introduced using the clots described herein. Promoters can be general promoters, yielding expression in a variety of mammalian cells, or cell specific, or even nuclear versus cytoplasmic specific. These are known to those skilled in the art and can be constructed using standard molecular biology protocols.

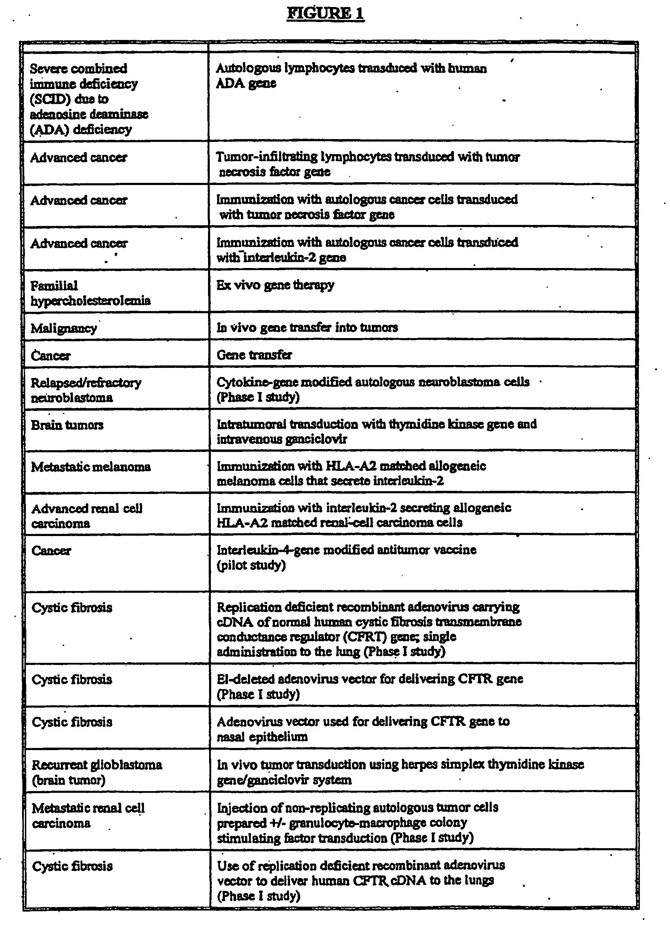

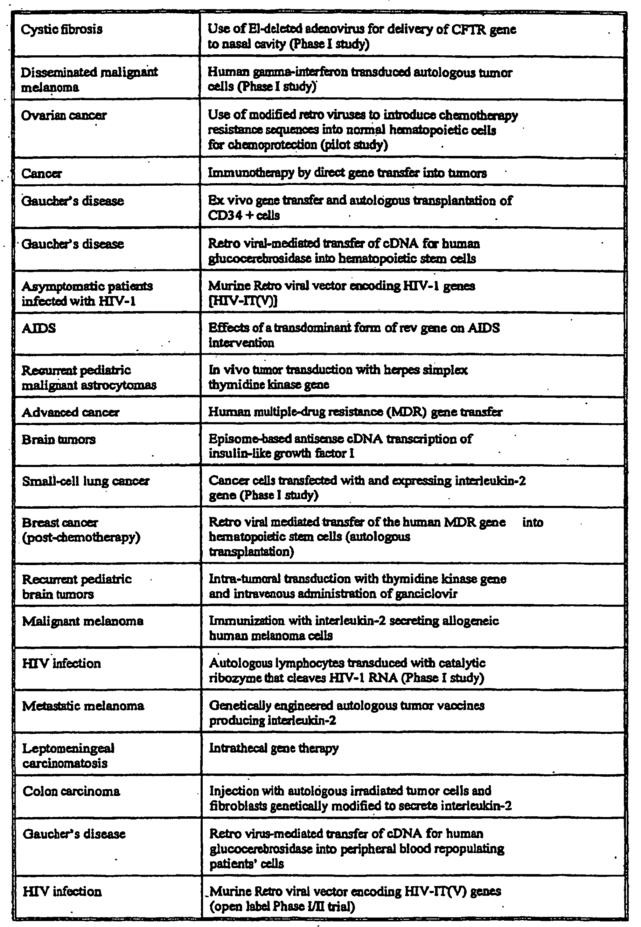

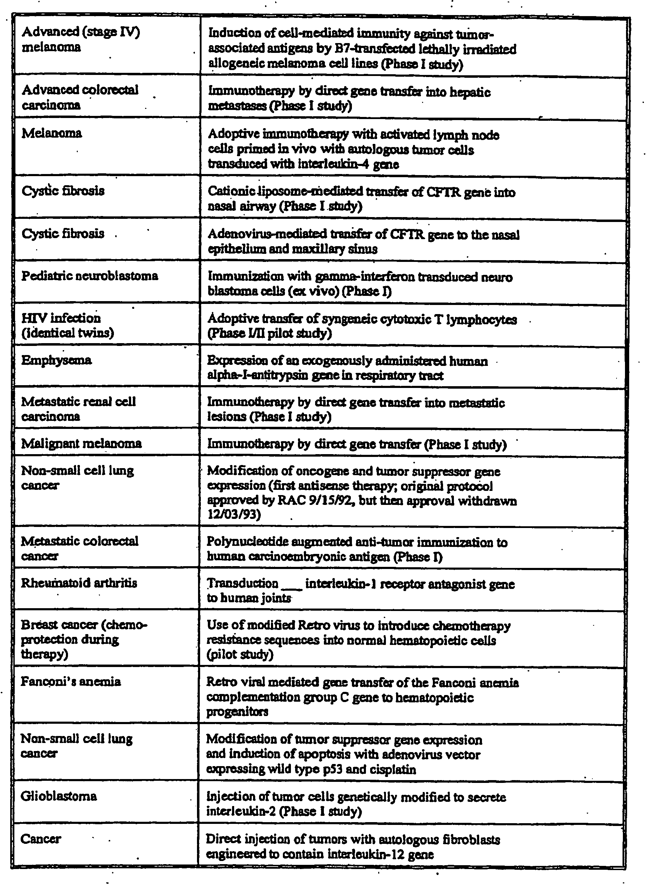

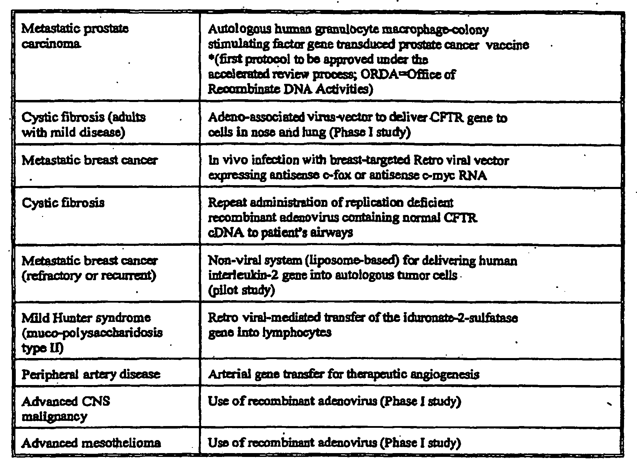

Any type of gene is useful according to the methods of the invention. The specific genes used in a particular circumstance will depend on the condition being treated and/or the desired therapeutic result. An exemplary list of genes that have been used in gene therapy is provided in Figure 1. In some embodiments of the invention, any one or combination of the genes listed in Figure 1 may be incorporated into the delivery device of the invention.

Gene transfer offers an approach that may overcome the many limitations of protein delivery to damaged tissues. The invention described in this disclosure presents a novel system for the application of gene delivery vehicles, cells and soluble proteins for the healing of damaged tissues. By delivering the cDNAs that code for proteins with reparative or therapeutic potential to specific cells at sites of injury or disease, the genetically-modified cells become local factors for drug production, permitting sustained synthesis of the specific protein.

Suitable promoters, enhancers, vectors, etc., for such genes are published in the literature. In general, useful genes replace or supplement function, including genes encoding missing enzymes such as adenosine deaminase (ADA) which has been used in clinical trials to treat ADA deficiency and cofactors such as insulin and coagulation factor VIII. Genes which affect regulation can also be administered, alone or in combination with a gene supplementing or replacing a specific function. For example, a gene encoding a protein which suppresses expression of a particular protein-encoding gene can be administered by the clots of the invention. Genes can be obtained or derived from a variety of sources, including literature references, Genbank, or commercial suppliers. They can be synthesized using solid phase synthesis if relatively small, obtained from deposited samples such as those deposited with the American Type Culture Collection, Rockville, MD or isolated de novo using published sequence information.

In addition to genes, the substance may be a short oligonucleotides such as antisense and ribozymes which are distinguished from genes by their length and function. Unlike such short oligonucleotides, genes encode protein and therefore will typically be a minimum of greater than 100 base pairs in length, more typically in the hundreds of base pairs.

As used herein, vectors are agents that transport the gene into a cell without degradation and include a promoter yielding expression of the gene in the cells into which it is delivered.

It will also be recognized that the genes in expression vectors may be transfected into host cells and cell lines, e.g., prokaryotic (e.g. *E. coli*), or eukaryotic (e.g. dendritic cells, B cells, CHO cells, COS cells, yeast expression systems and recombinant baculovirus expression in insect cells) *in vitro.* These cells may then be incorporated into the clots. Especially useful are mammalian cells such as human, mouse, hamster, pig, goat, primate, etc. They may be of a wide variety of tissue types, and include primary cells and cell lines. Specific examples include keratinocytes, peripheral blood leukocytes, bone marrow stem cells and embryonic stem cells. The expression vectors require that the pertinent gene sequence be operably linked to a promoter.

In some embodiments, a virus vector for delivering a gene is selected from the group consisting of adenoviruses, adeno-associated viruses, poxviruses including vaccinia viruses and attenuated poxviruses, Semliki Forest virus, Venezuelan equine encephalitis virus, retroviruses, Sindbis virus, and Ty virus-like particle. Examples of viruses and virus-like particles which have been used to deliver exogenous nucleic acids include: replication-defective adenoviruses (e.g. Xiang et al., Virology 219:220-227, 1996; Eloit et al., J. Virol. 7:5375-5381, 1997; Chengalvala et al., Vaccine 15:335-339, 1997), a modified retrovirus (Townsend et al., J Virol. 71:3365-3374, 1997), a nonreplicating retrovirus (Irwin et al., J. Virol. 68:5036-5044, 1994), a replication defective Semliki Forest virus (Zhao et al., Proc. Natl. Acad. Sci. USA 92:3009-3013, 1995), canarypox virus and highly attenuated vaccinia virus derivative (Paoletti, Proc. Natl. Acad. Sci. USA 93:11349-11353, 1996), non-replicative vaccinia virus (Moss, Proc. Natl. Acad. Sci. USA 93:11341-11348, 1996), replicative vaccinia virus (Moss, Dev. Biol. Stand. 82:55-63, 1994), Venzuelan equine encephalitis virus (Davis et al., J. Virol. 70:3781-3787, 1996), Sindbis virus (Pugachev et al., Virology 212:587-594, 1995), and Ty virus-like particle (Allsopp et al., Eur. J. Immunol 26:1951-1959, 1996). In preferred embodiments, the virus vector is an adenovirus or an alphavirus.

Another preferred virus for certain applications is the adeno-associated virus, a doublestranded DNA virus. The adeno-associated virus is capable of infecting a wide range of cell types and species and can be engineered to be replication-deficient. It further has advantages, such as heat and lipid solvent stability, high transduction frequencies in cells of diverse lineages, including hematopoietic cells, and lack of superinfection inhibition thus allowing multiple series of transductions. The adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

In general, other preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Adenoviruses and retroviruses have been approved for human gene therapy trials. In general, the retroviruses are replication-deficient (i.e. capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman Co., New York (1990) and Murry, E.J. Ed. "Methods in Molecular Biology," vol. 7, Humana Press, Inc., Cliffton, New Jersey (1991).

Preferably the foregoing nucleic acid delivery vectors: (1) contain exogenous genetic material that can be transcribed and translated in a mammalian cell, and (2) optionally may contain on a surface a ligand that selectively binds to a receptor on the surface of a target cell, such as a mammalian cell, and thereby gains entry to the target cell.

Various techniques may be employed for introducing nucleic acids of the invention into cells, depending on whether the nucleic acids are introduced *in vitro* or *in vivo* in a host. Such techniques include transfection of nucleic acid-CaPO₄ precipitates, transfection of nucleic acids associated with DEAE, transfection or infection with the foregoing viruses including the nucleic acid of interest, liposome mediated transfection, and the like. For certain uses, it may be preferred to target the nucleic acid to particular cells, especially if the clot will be implanted or administered at a distant site from the target cell. In such instances, a vehicle used for delivering a nucleic acid of the invention into a cell (e.g. a retrovirus, or other virus; a liposome) after release from the clot can have a targeting molecule attached thereto. For example, a molecule such as an antibody specific for a surface membrane protein on the target cell or a ligand for a receptor on the target cell can be bound to or incorporated within the nucleic acid delivery vehicle. Where liposomes are employed to deliver the genes, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like.

The substance may be in any state, such as a solution, solid, vector, gas or any other state that would enable the substance to mix with the hematopoeitic cell to form a clot.

For direct gene transfer, the harvested blood or bone marrow can be added to a solution containing a gene transfer vector (viral or non-viral) or a protein in an appropriately sized and shaped vessel or in any vessel that would allow the cell sample to mix with the substance. This mixture can be titrated using a pipette or any other device or system that would mix the substance with the cell sample.

For an ex vivo gene delivery approach, the hematopoeitic cell, e.g., blood or bone marrow aspirate can be mixed with a suspension of naïve or genetically modified cells with or without an additional vector. The cells are then incorporated into the clot and returned to the body.

The invention also encompasses products. The products are substance delivery systems according to claims 1 and 2. As used herein a "substance delivery system" is a non-polymeric hematopoeitic cell clot containing a substance such that the non-polymeric hematopoeitic cell clot can deliver the substance to a subject.

When administered, the compositions (non-polymeric hematopoeitic cell clot containing the substance) can be administered in pharmaceutically acceptable preparations. Such preparations may routinely contain pharmaceutically acceptable concentrations of salt, buffering agents, preservatives, compatible carriers and optionally other non-incorporated therapeutic agents.

The compositions can be administered by any conventional route, including injection or by gradual infusion over time. The administration may, for example, be direct injection or implantation, oral, intravenous, intraperitoneal, intramuscular, intracavity, intrapulmonary, mucosal (i.e. rectal, vaginal, ocular, dermal, intranasal, etc.), subcutaneous, aerosol, or transdermal. The administration may be systemic or local.

The compositions of the invention are administered in effective amounts. An "effective amount" is that amount of a composition that alone, or together with further doses, produces the desired response. The desired response, of course, will depend on the particular condition being treated and the type of cell or active agent being administered within the clot. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons. The compositions used in the foregoing methods preferably are sterile and contain an effective amount of the substance for producing the desired response in a unit of weight or volume suitable for administration to a patient.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

### Examples

In the examples, bone marrow clots are used according to the present invention. Blood clots are comparative examples.

### In Vitro:

### Example 1: Loading capacity of blood or bone marrow clots

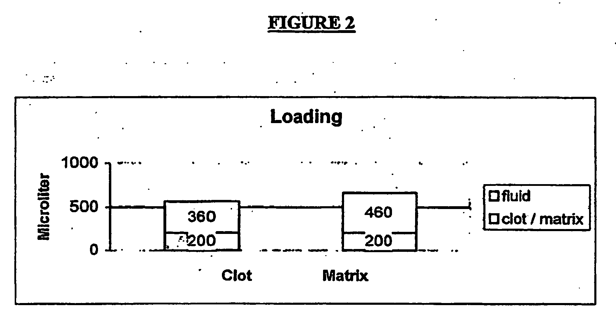

To evaluate the maximum amount of fluid that can be added to human blood without disrupting the clotting process, a 200 µl of volume of blood was mixed with increasing amounts of PBS. Clotting still occurred after adding a volume of PBS almost twice that of blood. As shown in Fig. 2, this finding was compared to the amount of fluid a collagen-glycosaminoglycan-matrix (collagen-gag-matrix) of the same size was able to absorb. No significant difference in the uptake of fluid between clots and collagen-gag-matrix was observed.

To determine whether a human blood clot is able to incorporate cells, rabbit bone marrow cells were cultured in monolayer and infected with an adenovirus vector carrying a gene encoding green fluorescent protein (GFP). After 24h, approximately 600,000 fluorescent cells were trypsinized and recovered by centrifugation. The cell pellets were each resuspended in 450 µl of human blood. These blood-cell constructs were then clotted in microcentrifuge tubes. After 1 h the clots were harvested, immersed in 1 ml of PBS and cultured for 24h in Ham's F12 medium, each in a 12 well plate.

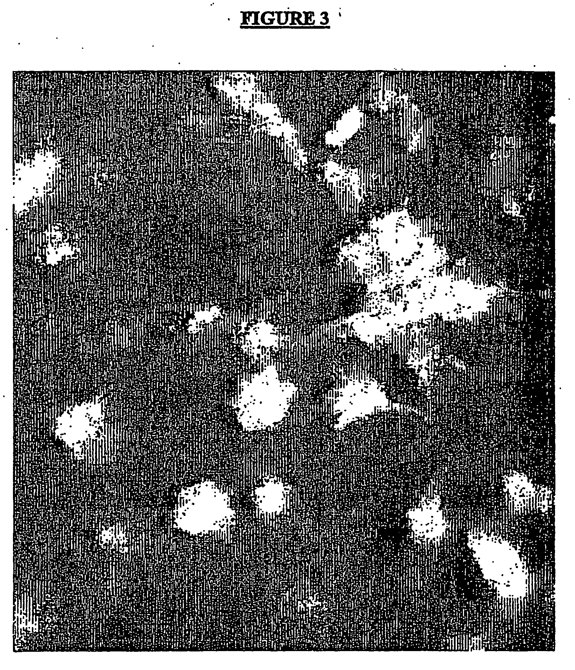

The human blood clots showed a high density of green fluorescent rabbit bone marrow cells 24 h after clotting, as can be seen in Fig. 3. Analysis of the remaining fluid in the Eppendorf tubes after clotting revealed no residual green cells, indicating that all of the transduced rabbit bone marrow cells had been retained by the human blood clots.

### Example 2: Form of the clot

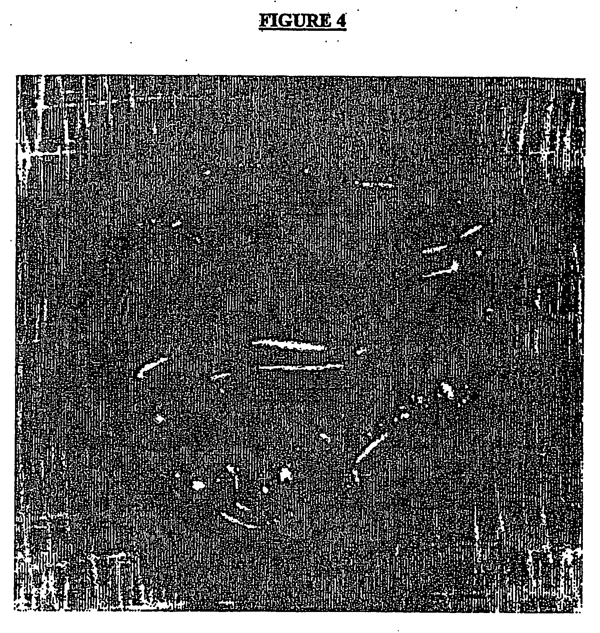

Experiments using different vessels for clotting, confirmed that clots could be formed into a wide variety of shapes and sizes. Clots thus generated remained stable enough to be implanted in any size and shape of defect, an example of which is shown in Fig. 4.

### Example 3: Seeding of blood clots and bone marrow clots with genetically modified cells (to simulate an ex vivo gene delivery approach)

To simulate an ex vivo gene delivery approach, 4 groups of rabbit blood clots were examined in vitro:
1. 450 µl rabbit blood only
2. 450 µl rabbit blood mixed with a suspension of 400,000 rabbit bone marrow cells
3. 450 µl rabbit blood mixed with a suspension of 400,000 rabbit bone marrow cells genetically modified with recombinant adenovirus to express GFP
4. 450 µl rabbit blood mixed with a suspension of 400,000 rabbit bone marrow cells genetically modified with recombinant adenovirus to express TGF-µ

Each group contained 4 replicates. Clots were examined by fluorescent microscopy at days 1,3,7,14, and 21. TGF-β expression was determined in clots seeded with cells transduced to express TGF-β by measuring TGF-β levels in the media using ELISA.

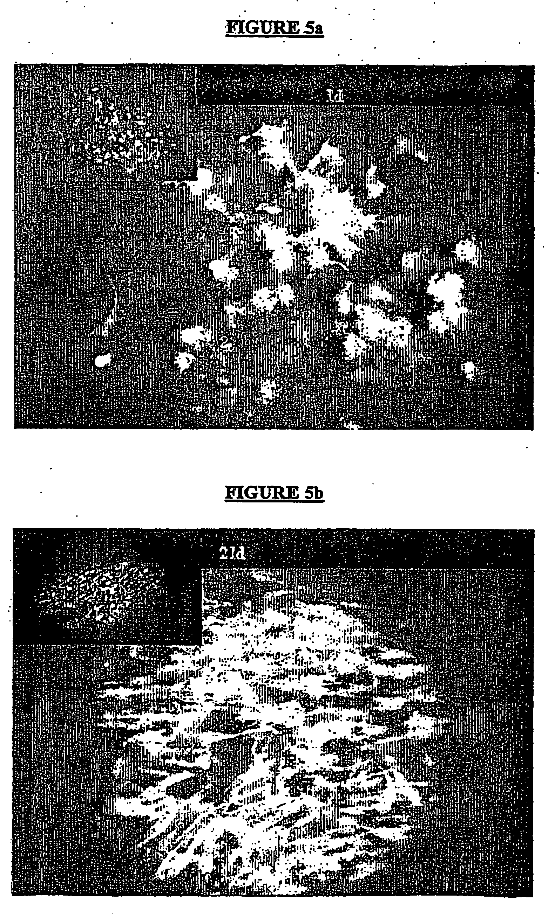

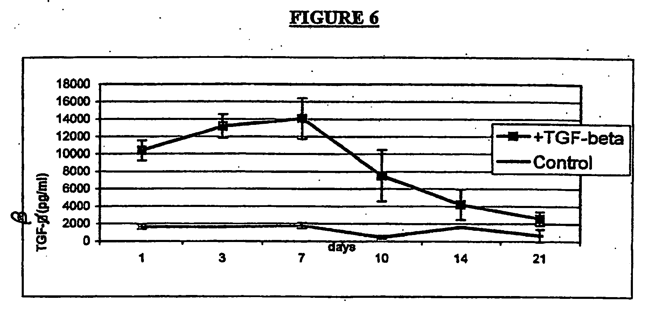

Fluorescent cells within clots were observed for at least 21 days in vitro. Fig. 5a shows the cells at day 1 and Fig. 5b shows the cells after 21 days. Similarly TGF-β expression was observed for at least 21 days with maximum expression at day seven; the results are shown in Fig. 6.

### Example 4: Direct transduction of cells within blood clots and bone marrow clots (to simulate the direct gene delivery approach)

To determine if cells within blood clots or bone marrow clots can support transgene expression, blood and bone marrow were mixed with the adenoviral vectors, Ad TGF-β and Ad GFP.
1. 450 µl rabbit blood only
2. 450 µl rabbit blood and 10 µl Ad GFP
3. 450 µl rabbit blood and 10 µl Ad TGF-β
4. 450 µl rabbit bone marrow-aspirate only
5. 450 µl rabbit bone marrow-aspirate and 10 µl Ad GFP
6. 450 µl rabbit bone marrow-aspirate and 10 µl Ad TGF-β

Each group consisted of 4 replicates. Clots were formed as described previously and were examined microscopically at day 1,3,7,14, and 21. ELISA of the conditioned media was performed at the same time points to measure TGF-β expression.

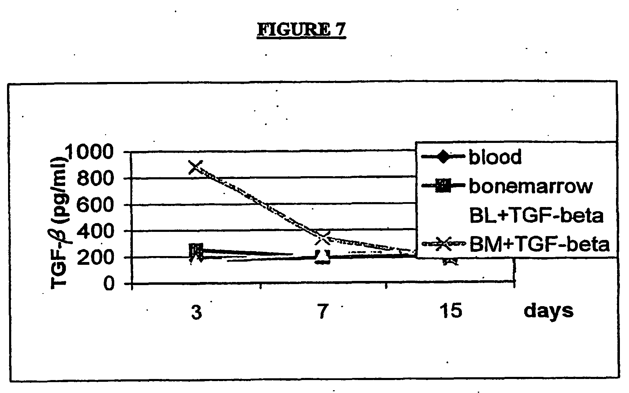

GFP expression was observed within blood clots and bone marrow clots for up to 14 days, the results of which can be seen in Fig. 7.

TGF-β production was detected for up to 7 days in bone marrow clots, with the maximum at day 3 and decreasing to background levels by day 14.

In contrast, detectable levels of TGF-β were not expressed in blood clots infected with Ad TGF-β. The absence of secreted TGF-β in the media may be due to the growth factor becoming trapped in the clot, or some other distinction with the vector.

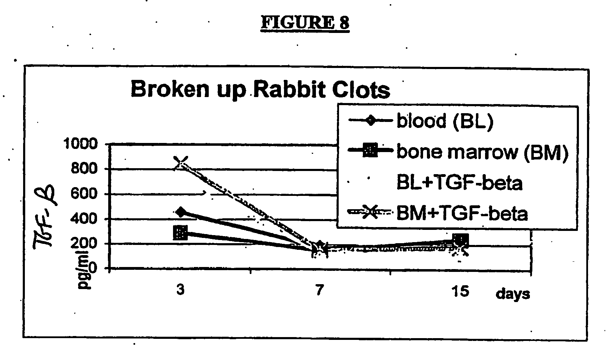

A further experiment to quantify these transgene products remaining trapped in the blood clots was performed using the same procedure, the results of which can be seen in Fig. 8.
1. Rabbit blood (450 µl)
2. Rabbit bone marrow (450 µl)
3. Rabbit blood (450 µl) and Ad TGF-β (10 µl)
4. Rabbit bone marrow (450 µl) and TGF-β(10 µl)

On the second day of culture, clots were harvested, washed in PBS, disaggregated mechanically, and cultured in Ham's F12 medium. TGF-β levels were assayed on days 3, 7, 14, 21.

TGF-β levels in the blood and bone marrow clots were high 3 days following gene delivery, but fell to very low levels by day 7. However, bone marrow clots showed a six fold higher total expression of TGF-β compared to blood clots which is likely due to their higher cellularity. As such the use of bone marrow seems to be more efficient for expression of transgene products following direct gene delivery than blood.

### Example 5: Stability of adenovirus in blood clots and bone marrow clots

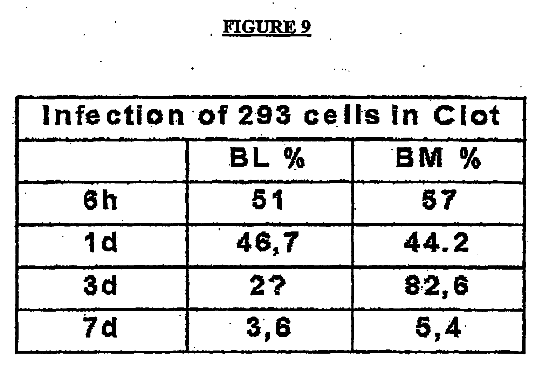

A further study was performed to determine if infectious viral vectors could be retained and remain transducing within clots, the results of which can be seen in Fig. 9.

Blood clots and bone marrow clots were infected with Ad GFP and cultured as described above. At various time points the clots were mechanically disaggregated and centrifuged to remove cell debris. The supernatants were collected and used to infect monolayer cultures of 293 cells. After 24 hours the cells were analyzed for fluorescence.

Fluorescent cells were indeed present in cultures that had been infected with the supernatants from broken up clots from days 1, 3, and 7.

This finding demonstrated the viral vector, Ad GFP, trapped in the clots, was able to retain its infectivity for at least 7 days in culture.

### In Vivo:

### Example 6: The use of autologous blood clots and bone marrow clots for direct gene delivery to cartilage defects in the knees of rabbits

For this objective, adenoviral gene delivery vectors encoding the genes for luciferase, GFP, and/or Lac Z were mixed and clotted with blood or bone marrow aspirate obtained from New Zealand white rabbits. After clotting (approximately 30 minutes) blood or bone marrow-vector constructs were implanted into surgically generated osteochondral defects in the femoral condyles of the same rabbits. Following implantation, the joint capsule was sutured and the animals revived. After day 3 the rabbits were sacrificed and the clots were harvested from the defects. For quantitative analyses, luciferase activity in the clot was determined. For qualitative analyses, fluorescent cells were viewed microscopically. The adjacent synovium was also examined for expression of the transgenes.

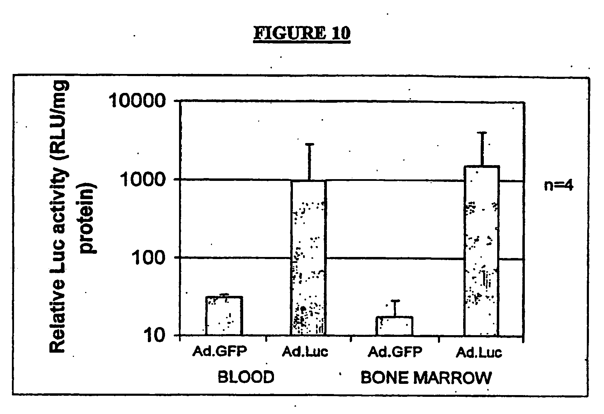

As shown in Fig. 10 high levels of luciferase transgene expression were observed in the harvested blood clots and bone marrow clots that had been mixed with Ad Luciferase. Similarly, large numbers of fluorescent cells were observed in the harvested clots that were infected with Ad GFP. A few green cells were also observed in the synovial lining immediately adjacent to the defect site. However, no expression was observed in other areas of the synovium. These results were in contrast to a highly green fluorescent synovial lining that is observed following direct gene delivery of a collagen-gag matrix containing Ad GFP to osteochondral defects. Thus, blood and bone marrow clots provide more contained localized transgene expression when implanted in vivo.

### Example 7: The potential for chondrogenesis or osteogenesis using rabbit blood clots and bone marrow clots

This study investigates the ability of endogenous precursor cells to change phenotype and undergo chondrogenic or osteogenic differentiation within blood and bone marrow clots. For this, blood and bone marrow were harvested from New Zealand white rabbits and clotted for "a total" of 6 groups:
1. Rabbit blood (450 µl) (1 clot)
2. Rabbit blood (450 µl) and Ad GFP (10 µl) (3 clots)
3. Rabbit blood (450 µl) and Ad TGF-β (10 µl) (4 clots)
4. Rabbit bone marrow (450 µl) (1 clot)
5. Rabbit bone marrow (450 µl) and GFP (10 µl) (5 clots)
6. Rabbit bone marrow (450 µl) and TGF-β (10 µl) (3 clots)

The clots were cultured in Ham's F12 medium for 6 weeks. After harvesting they were fixed, paraffin embedded, sectioned and examined for histology. The sections were stained with Hematoxylin-Eosin and Gomori's Trichrome Kit (Collagen Blue Staining). Sections were examined by three different individuals in a blinded manner.

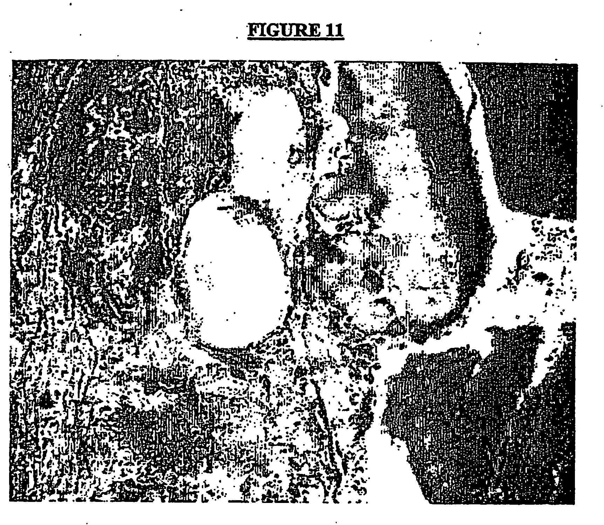

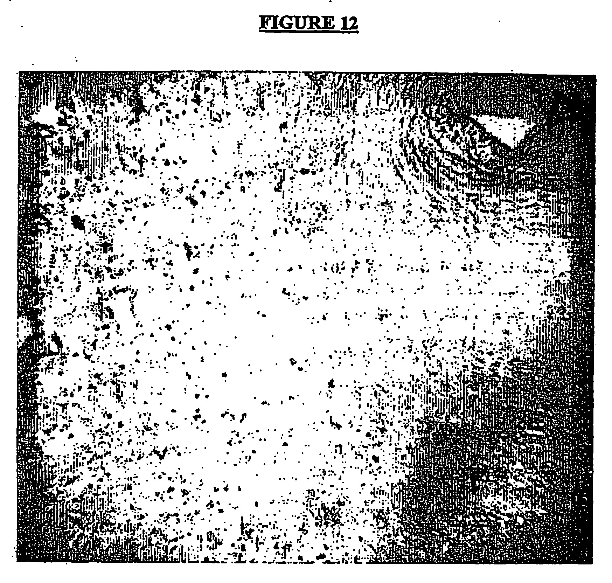

In bone marrow clots that were not genetically modified with growth factors (bone marrow only and bone marrow mixed with Ad GFP), the pluripotent nature of the endogenous cells was apparent. Areas of muscle-like, fat-like and fibrous tissue were found in all of these clots after 6 weeks, as depicted in Fig. 11. Bone marrow clots enriched with Ad TGF-β showed a more homogenous differentiation, with no muscle-like tissue seen. Instead, more fibrous tissue was observed, as depicted in Fig. 12.

Cell differentiation was not evident in blood clots. However, different concentrations and combinations of vectors may result in differentiation. These results suggest that cells within bone marrow clots are multi-potential, with the capacity to differentiate into many tissue types.

Because adenoviral vectors are powerful tools for studying the effects of over-expression of gene products, they were the vector of choice in the experiments described here. However, it is expected that other gene transfer vectors, such as Adeno Associated Virus (AAV) and retroviral vectors may have even greater clinical utility than vectors derived from adenoviruses.

### Example 8: The use of bone marrow clots modified with Ad.TGF-β1 for repairing cartilaginous tissue

This study investigates the use of bone marrow clots in repairing cartilaginous tissue. An osteochondral defect was created by drilling 3mm wide and 8mm deep holes through the cartilage and into the bone and marrow of rabbit knees. Control defects were either left untreated (empty defect) or received an unmodified bone marrow clot. A bone marrow clot, which was pre-infected with adenovirus containing the gene for transforming growth factor beta-1 (TGF-β1), was implanted into the remaining osteochondral defects.

Slides were taken six weeks after surgery and stained with hematoxylin-eosin (H&E) and toluidine blue. H&E is a common acid-base histologic stain; toluidine blue acts as an indicator of glycosaminoglycans (GAGs).

In the control defect that was left untreated, a fibrous repair formed that did not resemble the flanking cartilage. The other control defect that was treated with the unmodified bone marrow clot implant shows only a bony surface and no GAGs.

The defect that was treated with the pre-infected bone marrow clot that contained TGF-β1, had a chondrogenic appearance, showing a robust extracellular matrix. In most of the repair, the repair matrix was stained dark blue, indicating GAGs were present. The cells within the repair matrix resemble chondrocytes morphologically, but appear in clusters. Underneath the cartilage repair tissue was robust bone formation. Also, the cartilage layer was about the same depth as the flanking tissue.

These results suggest that although the repair tissue is not perfect, using this approach to gene delivery, the biology of the cells within a coagulated bone marrow aspirate can be influenced in a positive direction.

### SUMMARY STATEMENT - ASPECTS

The present disclosure also relates to:
Aspect 1. A method of delivering a substance to a subject, the method comprising:
   administering to a subject a non-polymeric hematopoeitic cell clot containing a substance to deliver the substance to the subject.
Aspect 2. The method according to aspect 1, wherein the non-polymeric hematopoeitic cell clot comprises bone marrow cells.
Aspect 3. The method according to aspect 1, wherein the non-polymeric hematopoeitic cell clot comprises blood cells.
Aspect 4. The method according to aspect 1, wherein the substance comprises a gene transfer vehicle.
Aspect 5. The method according to aspect 1, wherein the substance comprises additional cells.
Aspect 6. The method according to aspect 5, wherein the additional cells comprise genetically engineered cells.
Aspect 7. The method according to aspect 5, wherein the additional cells comprise naïve cells.
Aspect 8. The method according to aspect 1, wherein the substance comprises proteins.
Aspect 9. The method according to aspect 1, wherein the substance comprises recombinant proteins.
Aspect 10. The method according to aspect 1, wherein the substance comprises soluble proteins.
Aspect 11. The method according to aspect 1, wherein the substance comprises bioactive molecules.
Aspect 12. The method according to aspect 1, wherein the non-polymeric hematopoeitic cell clot is delivered into bone.
Aspect 13. The method according to aspect 1, wherein the non-polymeric hematopoeitic cell clot is delivered into soft tissues.
Aspect 14. The method according to aspect 1, wherein the non-polymeric hematopoeitic cell clot is delivered into at least one of cartilage, ligaments, tendons, meniscuses and invertebral discs.
Aspect 15. The method according to aspect 1, wherein the shape and size of the non-polymeric hematopoeitic cell clot is determined by a mold.
Aspect 16. The method according to aspect 1, wherein the non-polymeric hematopoeitic cell clot is homogenized with the substance.
Aspect 17. The method according to aspect 1, wherein the non-polymeric hematopoeitic cell clot is genetically modified to express at least one of growth factors and other gene products that facilitate tissue repair.
Aspect 18. The method according to aspect 1, wherein the non-polymeric hematopoeitic cell clot has a volume that is determined by the size of a tissue to be repaired.
Aspect 19. The method according to aspect 1, wherein the non-polymeric hematopoeitic cell clot is collected from a subject.
Aspect 20. The method according to aspect 2, wherein the bone marrow cells are harvested from iliac crests.
Aspect 21. The method according to aspect 2, wherein the bone marrow cells are harvested from osteochondral defects that expose underlying bone marrow.
Aspect 22. The method according to aspect 1, wherein the substrate is in the form of a solution.
Aspect 23. The method according to aspect 1, wherein the non-polymeric hematopoeitic cell clot containing substance is titrated.
Aspect 24. The method according to aspect 23, wherein the titration is performed using a pipette.
Aspect 25. The method according to aspect 1, wherein the non-polymeric hematopoeitic cell clot is mixed with a suspension of at least one of naïve and genetically modified cells, forming a cell suspension.
Aspect 26. The method according to aspect 25, wherein the cell suspension contains at least one gene vector.
Aspect 27. The method according to aspect 25, wherein the cell suspension contains no additional gene vectors.
Aspect 28. A method according to aspect 1, wherein the delivery is a slow, localized release of the substance from the non-polymeric hematopoeitic cell clot.
Aspect 29. A method according to aspect 1, wherein the non-polymeric hematopoeitic cell clot is shaped in a way to allow an effective delivery of the substance.
Aspect 30. A method according to aspect 1, further comprising regenerating tissue in the area of substance delivery.
Aspect 31. The method according to aspect 1, wherein the non-polymeric hematopoeitic cell clot produced from a sample of hematopoeitic cells which is allowed to clot for 15-30 minutes.
Aspect 32. The method according to aspect 1, wherein the non-polymeric hematopoeitic cell clot produced from a sample of hematopoeitic cells which is allowed to clot at room temperature.
Aspect 33. The method according to aspect 1, wherein the non-polymeric hematopoeitic cell clot produced from a sample of hematopoeitic cells which is placed in a vessel to clot.
Aspect 34. The method according to aspect 33, wherein the non-polymeric hematopoeitic cell clot is harvested from the vessel.
Aspect 35. The method according to aspect 1, wherein the non-polymeric hematopoeitic cell clot produced from a sample of hematopoeitic cells which is washed in a Phosphate Buffer Saline.
Aspect 36. The method according to aspect 1, wherein any unbound substance is removed from the non-polymeric hematopoeitic cell clot.
Aspect 37. A method of preparing a non-polymeric hematopoeitic cell clot substance delivery system, the method comprising:
   adding a substance to a sample of hematopoeitic cells; and
   allowing the sample of hematopoeitic cells containing the substance to form a non-polymeric hematopoeitic cell clot.
Aspect 38. A method according to aspect 37, wherein the non-polymeric hematopoeitic cell clot is implanted into a subject.
Aspect 39. A method according to aspect 37, wherein the substance is delivered to the subject.
Aspect 40. A method according to aspect 39, wherein the delivery is a slow, localized release of the substance from the non-polymeric hematopoeitic cell clot.
Aspect 41. A method according to aspect 37, wherein the non-polymeric hematopoeitic cell clot is shaped in a way to allow an effective delivery of the substance.
Aspect 42. A method according to aspect 37, wherein the non-polymeric hematopoeitic cell clot delivery system is used to regenerate tissue.
Aspect 43. A method according to aspect 37, wherein the sample of hematopoeitic cells is collected from a subject.
Aspect 44. A method according to aspect 37, wherein the non-polymeric hematopoeitic cell clot is formed in a vessel.
Aspect 45. A method according to aspect 44, further comprising harvesting the non-polymeric hematopoeitic cell clot from the vessel.
Aspect 46. A method according to aspect 37, further comprising washing the non-polymeric hematopoeitic cell clot.
Aspect 47. A method according to aspect 46, wherein any unbound substance is removed by washing.
Aspect 48. A method according to aspect 46, wherein the non-polymeric hematopoeitic cell clot is washed in a Phosphate Buffer Saline.
Aspect 49. The method according to aspect 37, wherein the non-polymeric hematopoeitic cell clot is allowed to clot for 15-30 minutes.
Aspect 50. The method according to aspect 37, wherein the non-polymeric hematopoeitic cell clot is allowed to clot at room temperature.
Aspect 51. The method according to aspect 37, wherein the hematopoeitic cells comprise bone marrow cells.
Aspect 52. The method according to aspect 37, wherein the hematopoeitic cells comprise blood cells.
Aspect 53. The method according to aspect 37, wherein the substance comprises a gene transfer vehicle.
Aspect 54. The method according to aspect 37, wherein the substance comprises additional cells.
Aspect 55. The method according to aspect 54, wherein the additional cells comprise genetically engineered cells.
Aspect 56. The method according to aspect 54, wherein the additional cells comprise *naïve* cells.
Aspect 57. The method according to aspect 37, wherein the substance comprises proteins.
Aspect 58. The method according to aspect 37, wherein the substance comprises recombinant proteins.
Aspect 59. The method according to aspect 37, wherein the substance comprises soluble proteins.
Aspect 60. The method according to aspect 37, wherein the substance comprises bioactive molecules.
Aspect 61. The method according to aspect 38, wherein the non-polymeric hematopoeitic cell clot is delivered into bone.
Aspect 62. The method according to aspect 38, wherein the non-polymeric hematopoeitic cell clot is delivered into soft tissues.
Aspect 63. The method according to aspect 38, wherein the non-polymeric hematopoeitic cell clot is delivered into at least one of cartilage, ligaments, tendons, meniscuses and invertebral discs.
Aspect 64. The method according to aspect 37, wherein the shape and size of the non-polymeric hematopoeitic cell clot is determined by a mold.
Aspect 65. The method according to aspect 37, wherein the non-polymeric hematopoeitic cell clot is homogenized with the substance.
Aspect 66. The method according to aspect 37, wherein the hematopoeitic cells are genetically modified to express at least one of growth factors and other gene products that facilitate tissue repair.
Aspect 67. The method according to aspect 37, wherein the non-polymeric hematopoeitic cell clot has a volume that is determined by the size of a tissue to be repaired.
Aspect 68. The method according to aspect 51, wherein the bone marrow cells are harvested from iliac crests.
Aspect 69. The method according to aspect 51, wherein the bone marrow cells are harvested from osteochondral defects that expose underlying bone marrow.
Aspect 70. The method according to aspect 37, wherein the substance is in the form of a solution.
Aspect 71. The method according to aspect 37, wherein the non-polymeric hematopoeitic cell clot containing substance is titrated.
Aspect 72. The method according to aspect 71, wherein the titration is performed using a pipette.
Aspect 73. The method according to aspect 37, wherein the hematopoeitic cells are mixed with a suspension of at least one of naïve and genetically modified cells, forming a cell suspension.
Aspect 74. The method according to aspect 73, wherein the cell suspension contains at least one gene vector.
Aspect 75. The method according to aspect 73, wherein the cell suspension contains no additional gene vectors.
Aspect 76. A substance delivery system comprising:
   a non-polymeric hematopoeitic cell clot having a substance incorporated therein.
Aspect 77. A substance delivery system according to aspect 76, wherein the non-polymeric hematopoeitic cell clot is shaped in a way to allow an effective delivery of the substance.
Aspect 78. The substance delivery system according to aspect 76, wherein the non-polymeric hematopoeitic cell clot comprises bone marrow cells.
Aspect 79. The substance delivery system according to aspect 76, wherein the non-polymeric hematopoeitic cell clot comprises blood cells.
Aspect 80. The substance delivery system according to aspect 76, wherein the substance comprises a gene transfer vehicle.
Aspect 81. The substance delivery system according to aspect 76, wherein the substance comprises additional cells.
Aspect 82. The substance delivery system according to aspect 81, wherein the additional cells comprise genetically engineered cells.
Aspect 83. The substance delivery system according to aspect 81, wherein the additional cells comprise *naïve* cells.
Aspect 84. The substance delivery system according to aspect 81, wherein the substance comprises proteins.
Aspect 85. The substance delivery system according to aspect 81, wherein the substance comprises recombinant proteins.
Aspect 86. The substance delivery system according to aspect 76, wherein the substance comprises soluble proteins.
Aspect 87. The substance delivery system according to aspect 76, wherein the substance comprises bioactive molecules.
Aspect 88. The substance delivery system according to aspect 76, wherein the non-polymeric hematopoeitic cell clot is formulated for delivery into bone.
Aspect 89. The substance delivery system according to aspect 76, wherein the non-polymeric hematopoeitic cell clot is formulated for delivery into soft tissues.
Aspect 90. The substance delivery system according to aspect 76, wherein the non-polymeric hematopoeitic cell clot is formulated for delivery into at least one of cartilage, ligaments, tendons, meniscuses and invertebral discs.
Aspect 91. The substance delivery system according to aspect 76, wherein the shape and size of the non-polymeric hematopoeitic cell clot is determined by a mold.
Aspect 92. The substance delivery system according to aspect 76, wherein the non-polymeric hematopoeitic cell clot is homogenized with the substance.
Aspect 93. The substance delivery system according to aspect 76, wherein the non-polymeric hematopoeitic cell clot is genetically modified to express at least one of growth factors and other gene products that facilitate tissue repair.
Aspect 94. The substance delivery system according to aspect 76, wherein the non-polymeric hematopoeitic cell clot has a volume that is determined by the size of a tissue to be repaired.
Aspect 95. The substance delivery system according to aspect 76, wherein the non-polymeric hematopoeitic cell clot is prepared from an isolated sample of hematopoeitic cells.
Aspect 96. The substance delivery system according to aspect 78, wherein the bone marrow cells are isolated from iliac crest bone marrow cells.
Aspect 97. The substance delivery system according to aspect 78, wherein the bone marrow cells are isolated from osteochondral defects that expose underlying bone marrow.
Aspect 98. The substance delivery system according to aspect 76, wherein the substance is at least one of naive and genetically modified cells.
Aspect 99. The substance delivery system according to aspect 76, wherein the non-polymeric hematopoeitic cell clot is prepared by the process of allowing a sample of hematopoeitic cells to clot for 15-30 minutes.
Aspect 100. The substance delivery system according to aspect 76, wherein the non-polymeric hematopoeitic cell clot is prepared by the process of allowing a sample of hematopoeitic cells to clot at room temperature.
Aspect 101. The substance delivery system according to aspect 76, wherein the non-polymeric hematopoeitic cell clot is prepared by the process of allowing a sample of hematopoeitic cells to clot in a vessel.
Aspect 102. The substance delivery system according to aspect 101, further comprising harvesting the non-polymeric hematopoeitic cell clot from the vessel.
Aspect 103. The substance delivery system according to aspect 76, wherein the non-polymeric hematopoeitic cell clot is prepared by the process of washing the non-polymeric hematopoeitic cell clot in a Phosphate Buffer Saline.
Aspect 104. The substance delivery system according to aspect 76, wherein the non-polymeric hematopoeitic cell clot is prepared by the process of removing any unbound substance from the non-polymeric hematopoeitic cell clot.

## Claims

1. A-delivery system comprising:
a non-polymeric bone marrow cell clot having an exogenous substance selected from the group consisting of a gene transfer vehicle, a protein, and a bioactive agent which has a prophylactic or therapeutic effect in a biological organism incorporated therein, wherein the non-polymeric bone marrow cell clot is
a) formulated for delivery into soft tissues, cartilage, ligaments, tendons, or meniscuses or intervertebral discs, or
b) made from iliac crest bone marrow cells, and
wherein a polymer matrix is not incorporated into the clot or used as the structure for the clot.

2. A delivery system consisting essentially of:
a non-polymeric bone marrow cell clot having an exogenous substance selected from the group consisting of a gene transfer vehicle, a protein, and a bioactive agent which has a prophylactic or therapeutic effect in a biological organism incorporated therein.

3. The delivery system of any of claims 1-2, wherein the exogenous substance is a bioactive agent which has a prophylactic or therapeutic effect in a biological organism.

4. The delivery system of claim 2, wherein the bone marrow cells are isolated from osteochondral defects that expose underlying bone marrow.

5. The delivery system of any of claims 1-4, wherein the non-polymeric bone marrow cell clot is formulated for delivery into at least one of cartilage, ligaments, tendons, meniscuses and intervertebral discs.

6. The delivery system of any of claims 1-5, wherein the non-polymeric bone marrow cell clot has a volume that is determined by the size of a tissue to be repaired.

7. The delivery system of any of claims 1-6, wherein the substance is formulated for delivery as a slow, localized release of the substance from non-polymeric bone marrow cell clot.

8. A composition for use in delivering an exogenous substance to a subject, comprising:
the delivery system of any one of claims 1-7 for the purpose of delivering the substance to a subject.

9. The composition of claim 8, wherein the delivery system is delivered to soft tissues.

10. The composition of claim 8, wherein the delivery system is delivered to cartilage.

11. The composition of claim 8, wherein the delivery system is delivered to ligaments.

## Patentansprüche

1. Verabreichungssystem umfassend:
einen nicht-polymeren Zellklumpen aus Knochenmarkzellen mit einer exogenen Substanz, die aus der Gruppe ausgewählt ist, die aus einem Gentransfervehikel, einem Protein und einem bioaktiven Wirkstoff besteht, der in einem biologischen Organismus, in den er eingefügt ist, eine prophylaktische oder therapeutische Wirkung besitzt, wobei der nicht-polymere Zellklumpen aus Knochenmarkzellen
a) zur Verabreichung in Weichgeweben, Knorpel, Bändern, Sehnen oder Menisken oder Bandscheiben formuliert ist oder
b) aus Knochenmarkzellen des Beckenkamms hergestellt wird, und
wobei keine polymere Matrix in den Klumpen eingefügt ist oder als der Träger für den Klumpen verwendet wird.

2. Verabreichungssystem, im Wesentlichen bestehend aus:
einem nicht-polymeren Zellklumpen aus Knochenmarkzellen mit einer exogenen Substanz, die aus der Gruppe ausgewählt ist, die aus einem Gentransfervehikel, einem Protein und einem bioaktiven Wirkstoff besteht, der in einem biologischen Organismus, in den er eingefügt ist, eine prophylaktische oder therapeutische Wirkung besitzt.

3. Verabreichungssystem nach einem der Ansprüche 1 bis 2, wobei es sich bei der exogenen Substanz um einen bioaktiven Wirkstoff handelt, der in einem biologischen Organismus eine prophylaktische oder therapeutische Wirkung besitzt.

4. Verabreichungssystem nach Anspruch 2, wobei die Knochenmarkzellen aus osteochondralen Defekten, bei denen das darunter liegende Knochenmark freigelegt wird, isoliert werden.

5. Verabreichungssystem nach einem der Ansprüche 1 bis 4, wobei der nicht-polymere Zellklumpen aus Knochenmarkzellen zur Verabreichung in mindestens eines aus Knorpel, Bänder, Sehnen, Menisken und Bandscheiben formuliert ist.

6. Verabreichungssystem nach einem der Ansprüche 1 bis 5, wobei der nicht-polymere Zellklumpen aus Knochenmarkzellen ein Volumen aufweist, das von der Größe eines zu reparierenden Gewebes bestimmt wird.

7. Verabreichungssystem nach einem der Ansprüche 1 bis 6, wobei die Substanz zur Verabreichung durch eine langsame, lokale Freigabe der Substanz aus dem nicht-polymeren Zellklumpen aus Knochenmarkzellen formuliert ist.

8. Zusammensetzung zur Verwendung bei der Verabreichung einer exogenen Substanz an einen Probanden, umfassend:
das Verabreichungssystem nach einem der Ansprüche 1 bis 7 zum Zweck der Verabreichung der Substanz an einen Probanden.

9. Zusammensetzung nach Anspruch 8, wobei das Verabreichungssystem an Weichgewebe verabreicht wird.

10. Zusammensetzung nach Anspruch 8, wobei das Verabreichungssystem an Knorpel verabreicht wird.

11. Zusammensetzung nach Anspruch 8, wobei das Verabreichungssystem an Bänder verabreicht wird.

## Revendications

1. Système de livraison comprenant :
un caillot cellulaire non polymère de moelle osseuse ayant une substance exogène sélectionnée parmi le groupe constitué d'un excipient de transfert génique, d'une protéine et d'un agent bioactif qui possède un effet prophylactique ou thérapeutique dans un organisme biologique incorporé en son sein, dans lequel le caillot cellulaire non polymère de moelle osseuse est
a) formulé pour la livraison dans des tissus mous, du cartilage, des ligaments, des tendons ou des ménisques ou des disques intervertébraux, ou
b) réalisé à partir de cellules de moelle osseuse de crête iliaque, et
dans lequel une matrice polymère n'est pas incorporée dans le caillot ou utilisée comme structure pour le caillot.

2. Système de livraison constitué essentiellement de :
un caillot cellulaire non polymère de moelle osseuse ayant une substance exogène sélectionnée parmi le groupe constitué d'un excipient de transfert génique, d'une protéine et d'un agent bioactif qui possède un effet prophylactique ou thérapeutique dans un organisme biologique incorporé en son sein.

3. Système de livraison selon l'une quelconque des revendications 1 et 2, dans lequel la substance exogène est un agent bioactif qui possède un effet prophylactique ou thérapeutique dans un organisme biologique.

4. Système de livraison selon la revendication 2, dans lequel les cellules de moelle osseuse sont isolées à partir de défauts ostéochondraux qui exposent la moelle osseuse sous-jacente.

5. Système de livraison selon l'une quelconque des revendications 1 à 4, dans lequel le caillot cellulaire non polymère de moelle osseuse est formulé pour la livraison dans au moins un élément parmi le cartilage, les ligaments, les tendons, les ménisques et les disques intervertébraux.

6. Système de livraison selon l'une quelconque des revendications 1 à 5, dans lequel le caillot cellulaire non polymère de moelle osseuse a un volume qui est déterminé par la taille d'un tissu devant être réparé.

7. Système de livraison selon l'une quelconque des revendications 1 à 6, dans lequel la substance est formulée pour la livraison en tant que libération localisée lente de la substance depuis le caillot cellulaire non polymère de moelle osseuse.

8. Composition destinée à être utilisée dans la livraison d'une substance exogène à un sujet comprenant :
le système de livraison selon l'une quelconque des revendications 1 à 7 dans le but de livrer la substance à un sujet.

9. Composition selon la revendication 8, dans laquelle le système de livraison est livré à des tissus mous.

10. Composition selon la revendication 8, dans laquelle le système de livraison est livré à du cartilage.

11. Composition selon la revendication 8, dans laquelle le système de livraison est livré à des ligaments.
